# EUROPEAN PATENT SPECIFICATION

(11) **EP 4 129 200 B1**
(45) Date of publication and mention of the grant of the patent: **26.08.2026**
(21) Application number: 21774074.5
(22) Date of filing: 12.02.2021
(51) Int. Cl.: A61B 5/16, A61B 5/00, A61B 3/113, G16H 10/20, G16H 30/40, G16H 50/20, G16H 50/30

(54) **COGNITIVE IMPAIRMENT DIAGNOSTIC DEVICE AND COGNITIVE IMPAIRMENT DIAGNOSTIC PROGRAM**
VORRICHTUNG ZUR DIAGNOSE KOGNITIVER BEEINTRÄCHTIGUNG UND PROGRAMM ZUR DIAGNOSE KOGNITIVER BEEINTRÄCHTIGUNG
DISPOSITIF DE DIAGNOSTIC DE DÉFICIENCE COGNITIVE ET PROGRAMME DE DIAGNOSTIC DE DÉFICIENCE COGNITIVE

(30) Priority: 27.03.2020 JP 2020059001
(43) Date of publication of application: 08.02.2023
(60) Divisional application: 25150857.8 / 4 527 294
(73) Proprietor: OSAKA UNIVERSITY, Suita-shi Osaka 565-0871 (JP)
(72) Inventor: TAKEDA, Shuko, Suita-shi, Osaka 565-0871 (JP); MORISHITA, Ryuichi, Suita-shi, Osaka 565-0871 (JP)
(74) Representative: KATZAROV S.A.
(86) International application number: PCT/JP2021/005216
(87) International publication number: WO 2021/192704

(56) References cited:
- WO-A1-2019/098173
- JP-A- 2015 177 953
- JP-A- 2017 158 866
- JP-A- 2019 171 022
- JP-A- 2019 171 022
- JP-A- 2020 025 849
- JP-A- 2020 032 077
- OYAMA AKANE ET AL: "Novel Method for Rapid Assessment of Cognitive Impairment Using High-Performance Eye-Tracking Technology", vol. 9, no. 1, 10 September 2019 (2019-09-10), pages 12932, XP055821562, Retrieved from the Internet <URL:http://www.nature.com/articles/s41598-019-49275-x> DOI: 10.1038/s41598-019-49275-x

## Description

### [Technical Field]

The present invention relates to a cognitive impairment diagnostic device and a cognitive impairment diagnostic program for diagnosis of cognitive impairment.

### [Background Art]

The conventional technologies relating to devices for diagnosis of cognitive impairment include the technologies as described below.

Patent literature (PTL) 1 and PTL 2 propose medical diagnostic devices utilizing line-of-sight detection. Such medical diagnostic devices enable an objective diagnosis of brain function-related disorder by detecting head movement and eye movement of a subject.

PTL 3 proposes a system for detecting eye movements of a subject for diagnosis of neurological disease.

PTL 4 and PTL 5 propose eye test charts for examining an eye disease such as retinal and optic nerve diseases or examining an optical nerve disorder caused by, for example, an intracranial disease.

PTL 6 through PTL 9 propose autism diagnosis support systems for diagnosis of autism of a subject, using an eye-gaze detection unit that includes at least an imaging camera.

PTL 10 proposes a cerebral disorder diagnosis support device that detects line of sight and the pupils of a subject to determine the possibility of the subject having a cerebral disorder.

PTL 11 proposes a diagnosis support device capable of effectively supporting diagnosis of a visual perception disorder.

PTL 12 proposes an eyeball movement measuring device and measuring method capable of detecting an initial motion time of eye movement even from a rough image.

PTL 13 proposes a cognitive impairment diagnostic device that enables a simple, low-cost, objective, quantitative, and versatile diagnosis.

PTL 14 describes a conventional cognitive impairment diagnostic device. In a configuration, the cognitive impairment assessment comprises first a step of displaying on a screen a plurality of windows wherein a person face appears in only one of the windows, followed by a step of displaying after a predetermined time period all the windows as empty windows without the person face being shown anymore.

### [Citation List]

### [Patent Literature]

[PTL 1] Japanese Unexamined Patent Application Publication No. 6-70884
[PTL 2] Japanese Unexamined Patent Application Publication No. 6-70885
[PTL 3] Japanese Unexamined Patent Application Publication (Translation of PCT Application) No. 2016-523112
[PTL 4] Japanese Patent No. 4560801
[PTL 5] Japanese Patent No. 4116354
[PTL 6] Japanese Patent No. 5926210
[PTL 7] Japanese Patent No. 5912351
[PTL 8] Japanese Patent No. 5761048
[PTL 9] Japanese Patent No. 5761049
[PTL 10] Japanese Patent No. 5817582
[PTL 11] Japanese Unexamined Patent Application Publication No. 2017-158866
[PTL 12] Japanese Unexamined Patent Application Publication No. 2017-176302
[PTL 13] WO2019/098173
[PTL 14] Japanese Unexamined Patent Application Publication No. 2019171022

### [Summary of Invention]

### [Technical Problem]

In general, a cognitive functioning test (neurophysiological examination) in the form of face-to-face interview has been used for diagnosis of cognitive impairment and dementia. The cognitive functioning test is a method in which an examiner asks a subject some questions for assessing the cognitive function, scores the answers to the questions that are given orally or in the form of writing of characters and drawing of graphics, and calculates a cognitive function score. This method has a problem, however, that the test is time consuming. For example, even the simplest mini-mental state exam (MMSE) takes about 15 to 30 minutes. The method also has a problem that a subject experiences a significant psychological stress due to the nature of the test that is conducted in the form of face-to-face interview. Another problem is that scores largely vary depending on the difference in the skills of interviewing examiners and their interpretation of the answers to the questions.

To solve the foregoing problems, the present inventors propose the cognitive impairment diagnostic device of PTL 13. A new cognitive impairment diagnostic device is further awaited.

The present invention aims to provide a cognitive impairment diagnostic device and a cognitive impairment diagnostic program that achieve a simple, low-cost, objective, quantitative, and versatile diagnosis of cognitive impairment, using a new diagnostic method.

### [Solution to Problem]

The cognitive impairment diagnostic device according to the present invention is defined in independent claim 1.

### [Advantageous Effects of Invention]

The cognitive impairment diagnostic device and the cognitive impairment diagnostic program according to the present invention achieve a simple, low-cost, objective, quantitative, and versatile diagnosis of cognitive impairment.

### [Brief Description of Drawings]

[FIG. 1A]

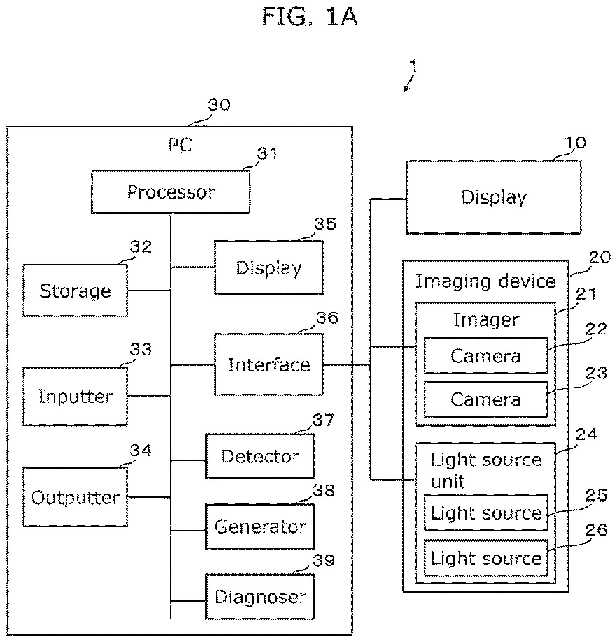

   FIG. 1A is a block diagram showing an example configuration of the cognitive impairment diagnostic device according to an embodiment.
[FIG. 1B]

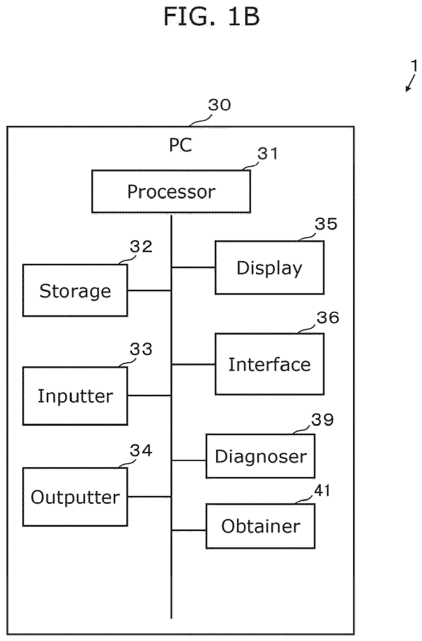

   FIG. 1B is a block diagram showing another example configuration of the cognitive impairment diagnostic device according to the embodiment.
[FIG. 2A]

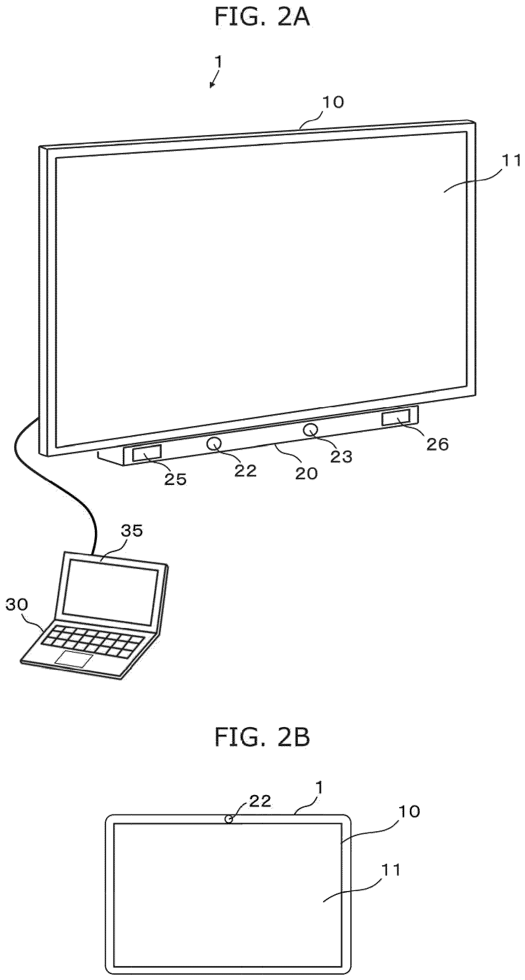

   FIG. 2A is a diagram showing an example external view of the cognitive impairment diagnostic device according to the embodiment.
[FIG. 2B]
   FIG. 2B is a diagram showing another example external view of the cognitive impairment diagnostic device according to the embodiment.
[FIG. 3]

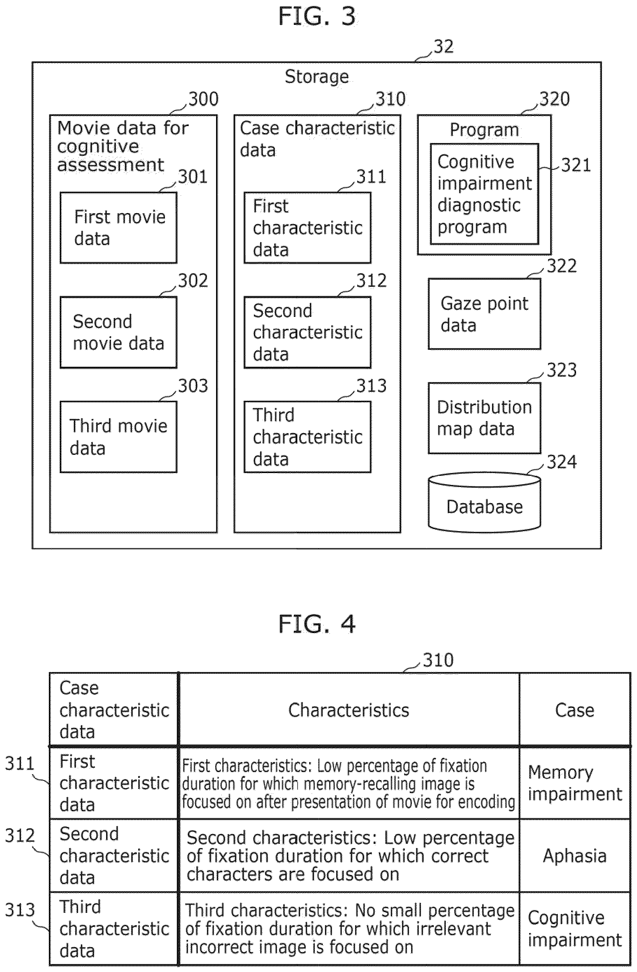

   FIG. 3 is a diagram showing an example of the storage content of storage according to the embodiment.
[FIG. 4]
   FIG. 4 is a diagram showing an example of case characteristics data according to the embedment.
[FIG. 5]

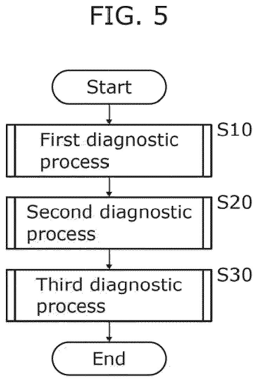

   FIG. 5 is a flowchart of example diagnostic processes performed by the cognitive impairment diagnostic device according to the embodiment.
[FIG. 6]

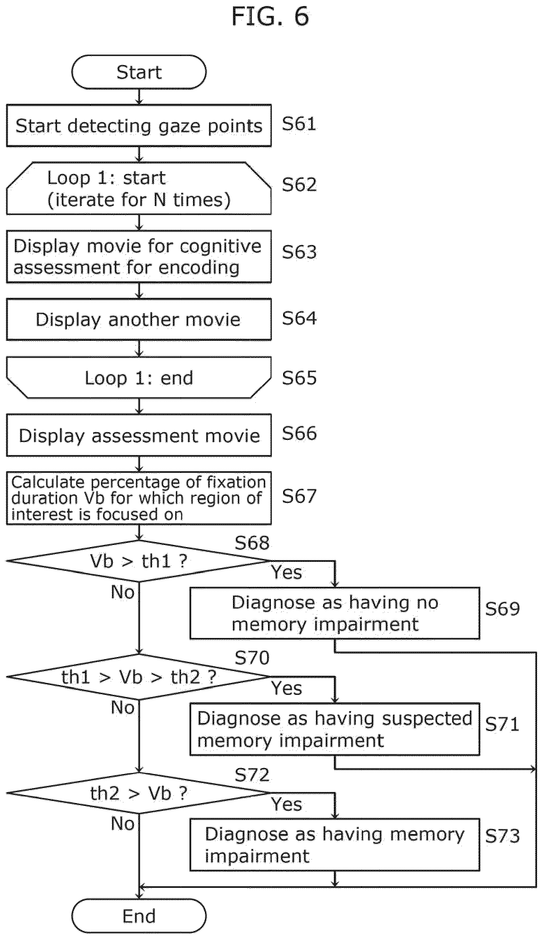

   FIG. 6 is a flowchart of an example first diagnostic process according to the embodiment.
[FIG. 7]

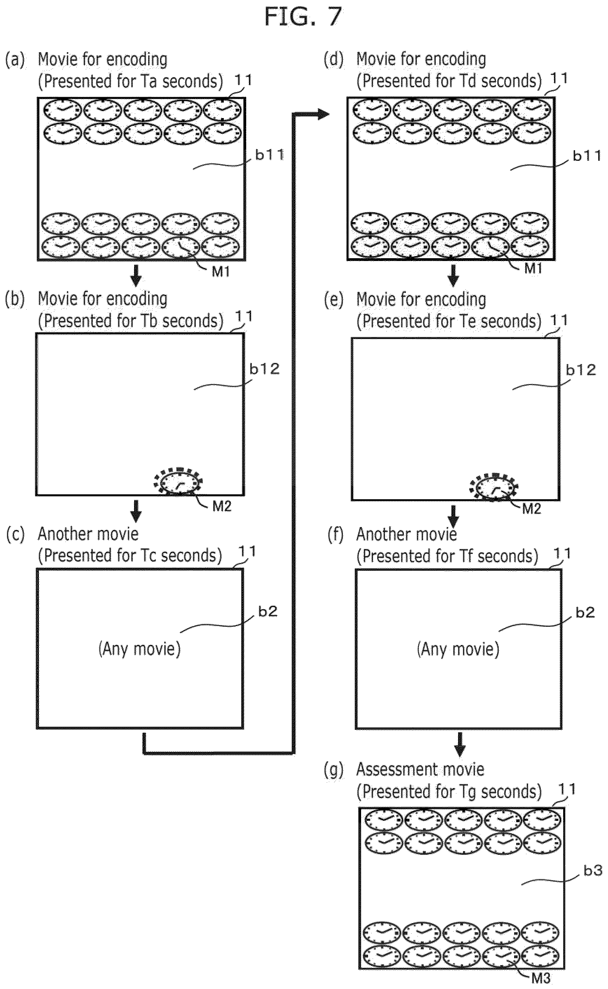

   FIG. 7 is a diagram showing an example movie for cognitive assessment and so forth in the first diagnostic process shown in FIG. 6.
[FIG. 8]

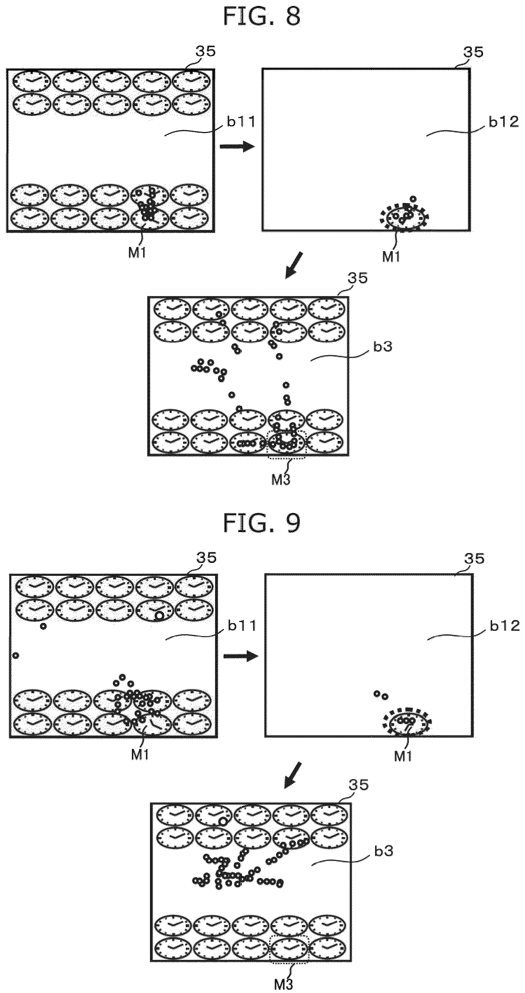

   FIG. 8 is a diagram showing an example distribution map obtained in the first diagnostic process performed on a subject without memory impairment.
[FIG. 9]
   FIG. 9 is a diagram showing an example distribution map obtained in the first diagnostic process performed on a subject with memory impairment.
[FIG. 10]

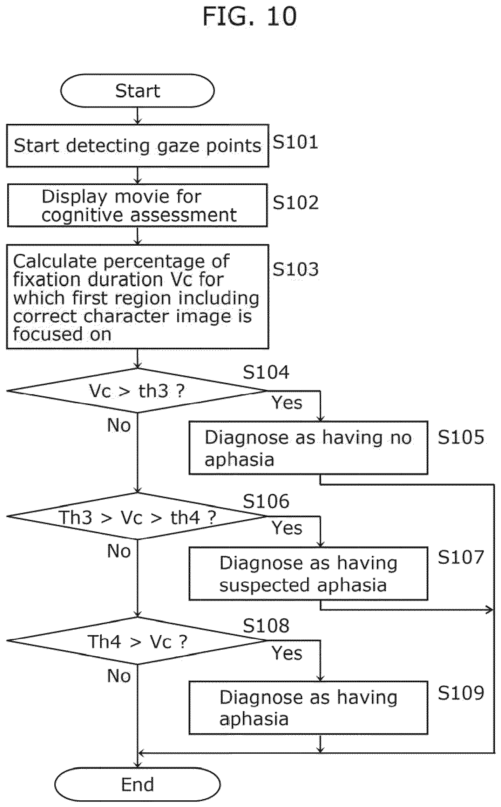

   FIG. 10 is a flowchart of an example second diagnostic process according to the embodiment.
[FIG. 11]

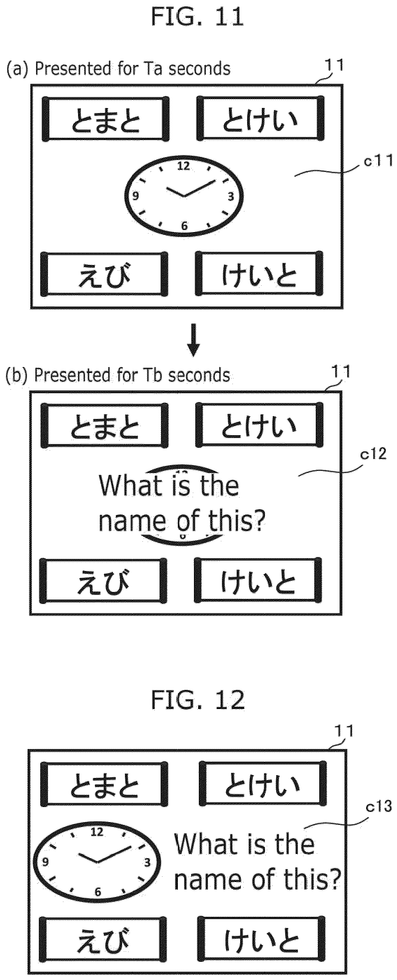

   FIG. 11 is a diagram showing an example movie for cognitive assessment in the second diagnostic process shown in FIG. 10 in which a linguistic instruction is used.
[FIG. 12]
   FIG. 12 is a diagram showing another example of the movie for cognitive assessment in the second diagnostic process shown in FIG. 10 in which a linguistic instruction is used.
[FIG. 13]

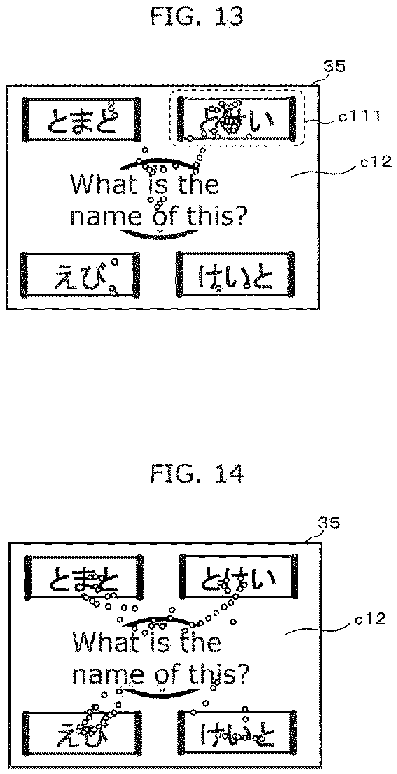

   FIG. 13 is a diagram showing an example distribution map obtained in the second diagnostic process that is performed, using FIG. 11, on a subject without aphasia.
[FIG. 14]
   FIG. 14 is a diagram showing an example distribution map obtained in the second diagnostic process that is performed, using FIG. 11, on a subject with aphasia.
[FIG. 15]

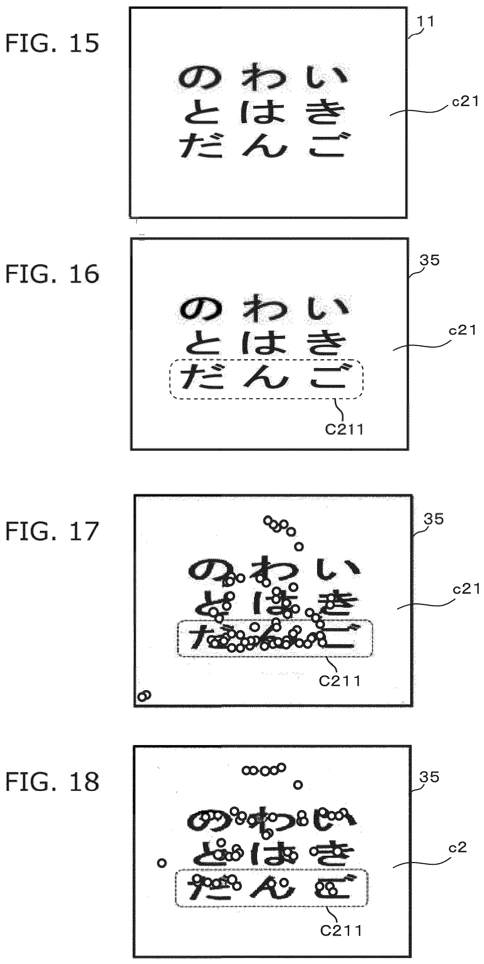

   FIG. 15 is a diagram showing a first example of a movie for cognitive assessment in the second diagnostic process shown in FIG. 10 in which no linguistic instruction is used.
[FIG. 16]
   FIG. 16 is a diagram showing an example assessment movie that corresponds to FIG. 15.
[FIG. 17]
   FIG. 17 is a diagram showing an example distribution map obtained in the second diagnostic process that is performed, using FIG. 15, on a subject without aphasia.
[FIG. 18]
   FIG. 18 is a diagram showing an example distribution map obtained in the second diagnostic process that is performed, using FIG. 15, on a subject with aphasia.
[FIG. 19]

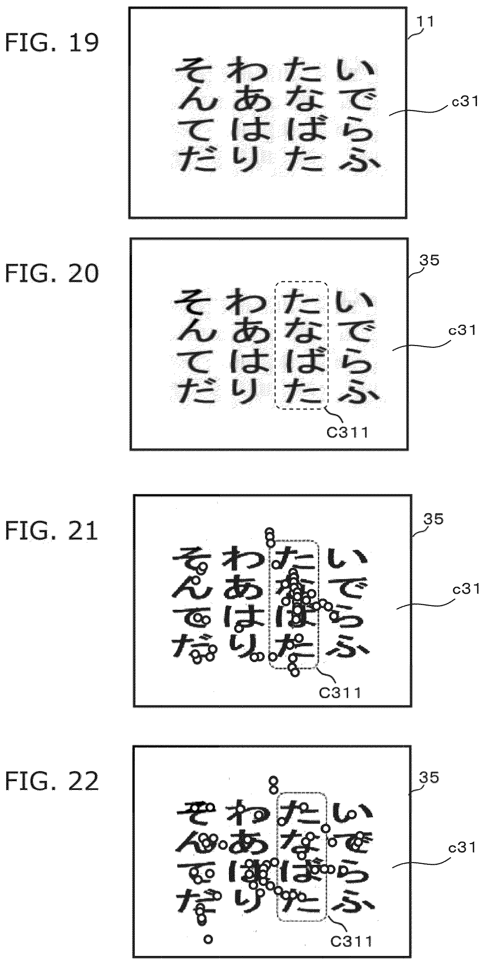

   FIG. 19 is a diagram showing a second example of a movie for cognitive assessment in the second diagnostic process shown in FIG. 10 in which no linguistic instruction is used.
[FIG. 20]
   FIG. 20 is a diagram showing an example assessment movie that corresponds to FIG. 19.
[FIG. 21]
   FIG. 21 is a diagram showing an example distribution map obtained in the second diagnostic process that is performed, using FIG. 20, on a subject without aphasia.
[FIG. 22]
   FIG. 22 is a diagram showing an example distribution map obtained in the second diagnostic process that is performed, using FIG. 20, on a subject with aphasia.
[FIG. 23]

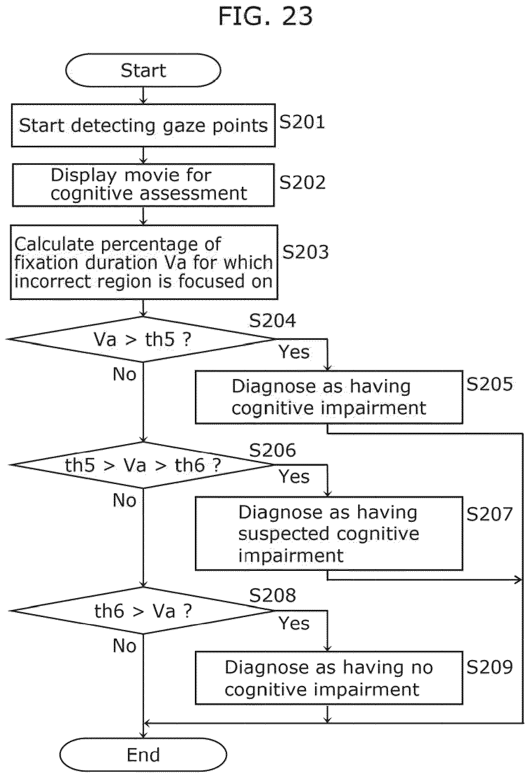

   FIG. 23 is a flowchart of an example third diagnostic process according to the embodiment.
[FIG. 24]

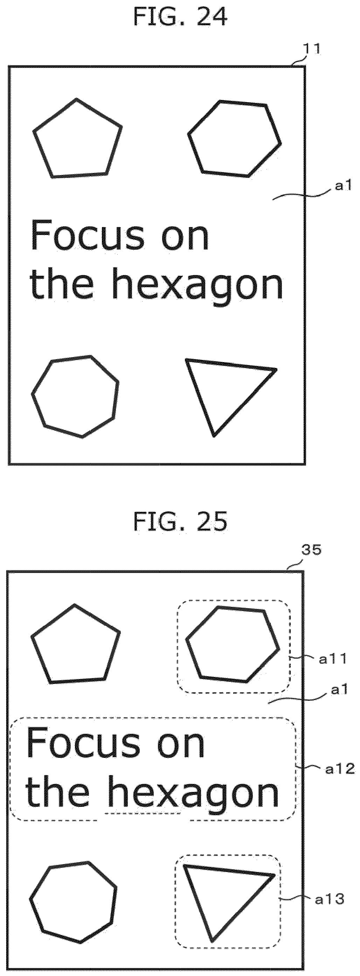

   FIG. 24 is a diagram showing an example movie for cognitive assessment used in the third diagnostic process shown in FIG. 23.
[FIG. 25]
   FIG. 25 is a diagram showing an example assessment movie used in the third diagnostic process shown in FIG. 23.
[FIG. 26]

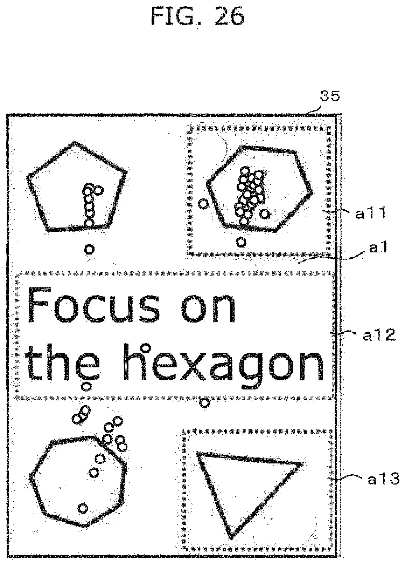

   FIG. 26 is a diagram showing an example distribution map in the third diagnostic process that is performed, using FIG. 24, on a subject without cognitive impairment.
[FIG. 27]

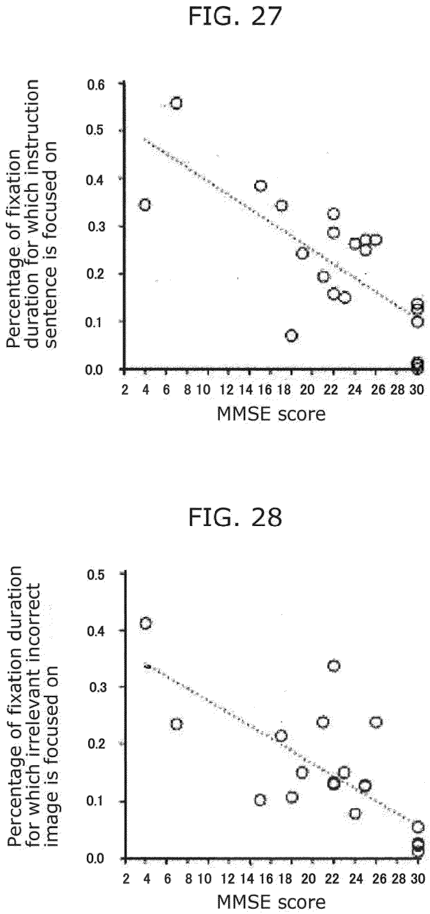

   FIG. 27 is a diagram showing a correlation between an MMSE score and the percentage of fixation duration for which an instruction sentence region in FIG. 25 is focused on in the third diagnostic process.
[FIG. 28]
   FIG. 28 is a diagram showing a correlation between an MMSE score and the percentage of fixation duration for which an irrelevant incorrect image in FIG. 25 is focused on in the third diagnostic process.

### [Description of Embodiment]

An embodiment will be specifically described below with reference to the drawings.

An exemplary embodiment described below shows a general or specific example. The numerical values, shapes, materials, elements, the arrangement and connection of the elements, steps, the processing order of the steps etc. shown in the following exemplary embodiment are mere examples, and therefore do not limit the scope of the appended Claims. Therefore, among the elements in the following exemplary embodiment, those not recited in any one of the independent claims are described as optional elements.

Also note that the drawings are schematic diagrams, and thus are not necessarily illustrated precisely. Also, the same elements are assigned the same reference marks throughout the drawings.

### [Embodiment]

The following describes the cognitive impairment diagnostic device and the cognitive impairment diagnostic program according to the embodiment with reference to the drawings.

### [1. Configuration of Cognitive Impairment Diagnostic Device]

FIG. 1A is a block diagram showing an example configuration of the cognitive impairment diagnostic device according to the embodiment. FIG. 2A is a diagram showing an example external view of the cognitive impairment diagnostic device according to the embodiment.

As shown in FIG. 1A, cognitive impairment diagnostic device 1 includes display 10, imaging device 20, and personal computer (PC) 30. An example configuration of cognitive impairment diagnostic device 1 is shown here in which a commercially available general PC 30 serves as a primary control device to which display 10 and imaging device 20 are further added.

Display 10 is a flat-panel display including display surface 11. Display 10 displays, on display surface 11, a movie for assessing cognitive impairment. For the purpose of showing the movie for cognitive assessment to a subject, display 10 is an oversize liquid-crystal display or organic electroluminescence display, as shown in FIG. 2A, that is easy even for the elderly to see. Display 10 may also be a PC monitor. Alternatively, a commercially available oversize television may serve as a monitor. Instead of a flat-panel display, display 10 may include a screen and a projector as display surface 11.

Imaging device 20 is a module attachable to display 10. Imaging device 20 includes at least imager 21 for capturing an image of the eyes of a subject and light source unit 24.

Imager 21 is a stereo camera including camera 22 and camera 23. Camera 22 and camera 23 may each be, for example, an infrared camera. Another example of camera 22 and 23 may each be a visible camera. Note that imager 21 may not be a stereo camera, and thus may be a single camera, or three or more cameras.

Light source unit 24 includes light source 25 and light source 26 for radiating an infrared ray to a subject as illumination light. Each of light source 25 and light source 26 may include, for example, one or more infrared light emitting diodes (LEDs). Another example may be light source 25 and light source 26 that include one or more white LEDs. Note that when illumination is sufficiently bright in the environment in which a subject is present, imaging device 20 may not include light source unit 24. Also, imaging device 20 may be attached to an upper portion of display 10 or separately attached on the right and left sides of display 10.

PC 30 includes processor 31, storage 32, inputter 33, outputter 34, display 35, interface 36, detector 37, generator 38, and diagnoser 39. Of the functional blocks shown in FIG. 1A, processor 31, storage 32, inputter 33, outputter 34, display 35, and interface 36 are implemented by general hardware and software products of a commercially available computer. The other functional blocks, i.e., detector 37, generator 38, and diagnoser 39, are structural elements that are mainly realized by processor 31 executing the cognitive impairment diagnostic program according to the present embodiment.

Processor 31 is what is known as a central processing unit (CPU) that executes a program stored in storage 32.

Storage 32 stores programs to be executed by processor 31 and data items to be processed by processor 31. Storage 32 further includes database 324. The programs stored in storage 32 include the cognitive impairment diagnostic program according to the present embodiment in addition to software products such as various firmware, an operating system (OS), and driver software. The data items stored in storage 32 include movie data for cognitive assessment, case characteristics data, gaze point data, distribution map data, etc. The movie data for cognitive assessment is still images or a moving image generated for diagnosis of cognitive impairment. The case characteristics data is data representing the characteristics of gaze point distributions that are typical of cognitive impairment. The gaze point data is time-series data indicating the positions and times of gaze points detected by detector 37. The distribution map is generated by generator 38 by sequentially plotting time-series gaze points in real time on a two-dimensional plane in accordance with the gaze point data. The distribution map shows a two-dimensional distribution of gaze points.

Note that storage 32 includes: a main memory or a primary memory that includes, for example, a dynamic random access memory (DRAM); an auxiliary memory or a secondary memory that includes, for example, a hard disc drive (HDD) device and a solid state drive (SSD) device; and a cache memory. Stated differently, the present description uses storage 32 as a generic name of the structural elements capable of storing programs and data.

Inputter 33 includes, for example, a keyboard, a mouse, a tack pad, etc. Inputter 33 receives an operation of an operator.

Outputter 34, an example of which is a speaker, outputs sound.

Display 35 is, for example, a liquid-crystal display. Display 35 displays, for example, a movie for cognitive assessment on which a distribution map is superimposed for monitoring by the user (here, examiner).

Interface 36 is capable of performing communications via a cable, with display 10 and imaging device 20 connected. Interface 36 includes, for example, a high-definition multimedia interface (HDMI)^{®} port and a universal serial bus (USB) port. In this case, interface 36 connects to display 10 via an HDMI^{®} cable and connects to imager 21 and light source unit 24 via a USB cable.

Detector 37 detects the gaze points of a subject on display surface 11 on the time series, on the basis of images captured by imager 21. For example, detector 37 detects the eye gaze of the subject from an image captured by imager 21 and detects, as the position of a gaze point, the coordinates of the point at which the eye gaze intersects with display surface 11. The positions of gaze points are detected at regular time intervals. The regular time intervals may be set between several 10 mS and several 100 mS, and thus may be, for example, 100 mS. Detector 37 generates, in real time, a group of coordinate data items (x, y, t) that include time as gaze point data representing the position of a time-series gaze point. Here, "x" and "y" are coordinates on a plane (e.g., display surface 11 or movie for cognitive assessment) and "t" is time.

Generator 38 generates a distribution map that represents the distribution of the gaze points detected by detector 37. The distribution map is, for example, a map on which marks (e.g., colored dots) corresponding to the foregoing coordinate data (x, y, t) are plotted on a two-dimensional plane. The distribution map is superimposed, in real time, on the movie for cognitive assessment that is displayed on display 35 of PC 30. Such marks may be displayed in a manner, for example, that a newer gaze point is brighter.

Diagnoser 39 makes diagnosis of the cognitive function of the subject by determining whether the distribution map has the characteristics of those indicated by the case characteristics data.

As described above, cognitive impairment diagnostic device 1 includes: display 10 that displays, on display surface 11, a movie for assessing cognitive impairment; imager 21 that captures an image of the eyes of a subject; detector 37 that detects, on the time series, gaze points of the subject on display surface 11, on the basis of the image captured by imager 21; generator 38 that generates a distribution map representing a distribution of the gaze points detected by detector 37; storage 32 that stores case characteristic data 310 indicating the characteristics of the gaze point distribution that are typical of cognitive impairment; and diagnoser 39 that makes diagnosis of cognitive impairment of the subject by determining whether the distribution map has the characteristics indicated by case characteristics data 310.

With the foregoing configuration, it is possible to enable cognitive impairment diagnostic device 1 to make a simple, low-cost, objective, quantitative, and versatile diagnosis of cognitive function.

Note that PC 30 shown in FIG. 1A and FIG. 2A may be any one of a notebook computer, a tablet computer, or a desktop computer.

The following describes another example configuration of cognitive impairment diagnostic device 1.

FIG. 1B is a block diagram showing another example configuration of cognitive impairment diagnostic device 1 according to the embodiment. FIG. 1B is different from FIG. 1A in that display 10, imager 20, detector 37, and generator 38 are deleted, and that obtainer 41 is added. The following avoids overlapping description of the common points and mainly describes the differences.

Display 10, imager 20, detector 37, and generator 38 in FIG. 1A correspond to the elements for eye tracking for generating distribution map data that represents the distribution of gaze points of a subject on the movie for cognitive assessment. In an example configuration shown in FIG. 1B, major elements for diagnosis of cognitive impairment are shown, with the elements for generating the distribution map data separated from FIG. 1A.

Obtainer 41 obtains the distribution map data generated by an external device, and stores it in storage 32. Such distribution map data is subjected to diagnosis of cognitive impairment.

Note that obtainer 41 is simply required to be capable of obtaining, from an external device, distribution map data, movie data for cognitive assessment, case characteristics data, and gaze point data. When movie data for cognitive assessment, case characteristics data, etc. that correspond to the distribution map data are already stored in storage 32, for example, obtainer 41 may obtain only the distribution map data. Also, for example, obtainer 41 may obtain, for the first subject, distribution map data, movie data for cognitive assessment, case characteristics data, and gaze point data corresponding to such subject, and obtain only distribution map data for the second and subsequent subjects.

Note that cognitive impairment diagnostic device 1 shown in FIG. 1A may further include obtainer 41 shown in FIG. 1B.

Also, the external device is simply required to be capable of generating a distribution map that represents the distribution of time-series gaze points of a subject by eye tracking, and thus an eye tracking method and a configuration for generating a distribution map are not limited to specific ones. Also, the external device may be connected to cognitive impairment diagnostic device 1 via the Internet, a phone line, or a dedicated line.

Note that cognitive impairment diagnostic device 1 is not required to be a device having oversize display 10 as shown in an example external view in FIG. 2A, and thus may be configured as a smart device.

FIG. 2B is a diagram showing another example external view of cognitive impairment diagnostic device 1. Cognitive impairment diagnostic device 1 in this diagram is a tablet smart device having the same functional structural elements as those shown in FIG. 1A, such as display 10 and imager 22.

Display 10 shown in FIG. 2B doubles as display 10 and display 35 of PC 30 shown in FIG. 2A. A touch panel is provided on a surface of display surface 11.

Imager 22 is a front camera of the smart device. Imager 22 is used, for example, for eye tracking by detecting eye positions and movements by a face recognition technology. In this case, light source unit 24 may not be included. Movie display for a subject is performed on display 10. Also, in accordance with a touch panel operation performed by the examiner, the cognitive impairment diagnostic program is executed by the processor inside of the tablet device. The smart device makes diagnosis of cognitive impairment by being alternately used by the subject and the examiner or by the examiner showing display 10 to the subject.

Cognitive impairment diagnostic device 1 as the foregoing smart device may be implemented, for example, by a tablet terminal, a smartphone, or a notebook PC. Also, the cognitive impairment diagnostic program is prepared as an app of these devices. Also note that cognitive impairment diagnostic device 1 shown in FIG. 2B may have the same functional structural elements as those shown in FIG. 1B.

### [1.1 Program and Data in Storage 32]

The following describes programs and data stored in storage 32.

FIG. 3 is a diagram showing an example of the storage content in storage 32 according to the embodiment. In the diagram, storage 32 stores movie data for cognitive assessment 300, case characteristics data 310, program 320, gaze point data 322, and distribution map data 323. Program 320 includes cognitive impairment diagnostic program 321. Storage 32 further includes database 324.

Movie data for cognitive assessment 300 is a set of movie data items including first movie data 301 through third movie data 303. Each of the movie data items is a movie generated for diagnosis of the presence or absence of cognitive impairment or the level of cognitive impairment, or a movie generated for distinguishing cases of cognitive impairment.

Case characteristics data 310 is data that represents the characteristics of gaze point distributions that are typical of cognitive impairment. Case characteristics data 310 is a set of characteristic data items including first characteristics data 311 through third characteristics data 313. First characteristics data 311 through third characteristics data 313 correspond to first movie data 301 through third movie data 304, respectively.

Program 320 includes software products, such as various firmware, an operating system (OS), and driver software, and cognitive impairment diagnostic program 321. Cognitive impairment diagnostic program 321 is a program executed by a computer, i.e., PC 30. Cognitive impairment diagnostic program 321 causes the computer to execute: displaying, on display surface 11, a movie for cognitive assessment for cognitive impairment; capturing, by imager 32, an image of the eyes of a subject; detecting, on the time series, gaze points of the subject on display surface 11, on the basis of the image captured by imager 21; generating a distribution map representing a distribution of the detected gaze points; and making diagnosis of cognitive impairment of the subject by determining whether the distribution map has the characteristics indicated by case characteristics data. Of these, the detecting, performed by PC 30, of the gaze points of the subject on display surface 11 on the time series, on the basis of the image captured by imager 21 is a function of detector 37. The generating, performed by PC 30, of the distribution map that represents the distribution of the detected gaze points is a function of generator 38. The making of diagnosis, performed by PC 30, of cognitive impairment of the subject by determining whether the distribution map has the characteristics indicated by the case characteristics data is a function of diagnoser 39.

Gaze point data 322 is time-series data representing the positions and times of the gaze points detected by detector 37. Gaze point data 322 is, for example, a set of coordinate data (x, y, t) that includes time as described earlier.

Distribution map data 323 is data that represents the distribution map as described earlier.

Database 324 stores various data items and others. Note that database 324 may not be a part of storage 32, and thus may be provided outside of storage 32. Database 324 may also be connected via a network such as the Internet.

Note that in addition to the program and the data items shown in FIG. 3, storage 32 also stores diagnostic data indicating the diagnostic result of each subject, and data that associates, with one another, gaze point data 322, distribution map data 323, and the diagnosis data of each subject.

Next, specific examples of case characteristics data 310 will be described.

FIG. 4 is a diagram showing an example of case characteristics data 310 according to the embodiment. Case characteristics data 310 shown in this diagram includes first characteristics data 311 through third characteristics data 313. FIG. 4 shows the respective characteristics of first characteristics data 311 through third characteristics data 313 and their corresponding cases of cognitive impairment.

First characteristics data 311 associates the first characteristics and memory impairment. The first characteristics are typical characteristics of a person with memory impairment. More specifically, the first characteristics indicate the characteristics that the percentage of fixation duration (hereinafter also referred to as "% fixation duration") for which a memory-recalling image is focused on is low on a distribution map of gaze points when such memory-recalling image is presented to a person with memory impairment after the presentation of a movie for encoding. A precondition of the first characteristics is that a movie for encoding, which is the movie for cognitive assessment of first movie data 301, is presented to a subject, after which a memory-recalling image is further presented to the subject.

Second characteristics data 312 associates the second characteristics and aphasia. Stated differently, the second characteristics are typical characteristics of a person with aphasia. More specifically, the second characteristics indicate the characteristics that the % fixation duration for which a correct image is focused on is low on a distribution map of gaze points in an image that includes a plurality of alternatives including the correct image and an incorrect image. A precondition of the second characteristics is that a second movie, which is the movie for cognitive assessment of second movie data 302, is being presented to a subject.

Third characteristics data 313 associates the third characteristics and cognitive impairment. Stated differently, the third characteristics are typical characteristics of a person with cognitive impairment. More specifically, the third characteristics indicate the characteristics that the % fixation duration for which an incorrect image is focused on is no small on a distribution map of gaze points in an image including a plurality of alternatives including a correct image and the incorrect image. A precondition of the third characteristics is that a third movie, which is the movie for cognitive assessment of third movie data 303, is being presented to a subject.

### [1.2 Example Processes of Cognitive Impairment Diagnostic Device 1]

The following describes example processes performed by cognitive impairment diagnostic device 1 according to the embodiment with the foregoing configuration.

FIG. 5 is a flowchart of example diagnostic processes performed by cognitive impairment diagnostic device 1 according to the embodiment.

As shown in FIG. 5, cognitive impairment diagnostic device 1 sequentially performs a first diagnostic process (S10) through a third diagnostic process (S30). Example diagnostic processes shown in FIG. 5 are processes mainly realized by PC 30 executing cognitive impairment diagnostic program 321.

The first diagnostic process is a diagnostic process that uses first movie data 301 and first characteristics data 311. The second diagnostic process is a diagnostic process that uses second movie data 302 and second characteristics data 312. The third diagnostic process is a diagnostic process that uses third movie data 303 and third characteristics data 313.

The duration of each of the first diagnostic process through the third diagnostic process is on the order of 0.5 minutes to a few minutes.

Note that the order of the first diagnostic process through the third diagnostic process may be different from the one shown in FIG. 5.

In FIG. 5, at least one of the first diagnostic process through the third diagnostic process may be selected and performed.

Note that a calibration process for gaze point detection may be performed before starting the example diagnostic processes shown in FIG. 5.

### [2.1 First Diagnostic Process]

The following describes in detail the first diagnostic process in step S10 shown in FIG. 5. The first diagnostic process uses the foregoing first characteristics to make diagnosis of memory impairment.

FIG. 6 is a flowchart of an example of the first diagnostic process (S10) according to the embodiment. FIG. 7 is a diagram showing an example movie for cognitive assessment and so forth used in the first diagnostic process shown in FIG. 6.

As shown in FIG. 6, PC 30 starts the detection of gaze points by detector 37 (S61), and executes loop 1 (S62 through S65), which is processing for causing a subject to remember the region of a specific image or characters.

In loop 1, PC 30 displays a movie for cognitive assessment for encoding (S63), and displays another movie immediately after that (S64). N, which is the number of iterating loop 1, may be 2 or more.

In step S63, the movie for encoding is presented that induces the eye gaze of the subject to unconsciously focus on a specific graphic or region on the screen, that is, without an intervention of a linguistic instruction. In an example shown in FIG. 7, the movie for cognitive assessment includes movie for encoding b11 and movie for encoding b12 in time order. Movie for encoding b11 is displayed for Ta seconds, immediately after which movie for encoding b12 is displayed for Tb seconds. For example, time Ta may be on the order of five seconds and time Tb may be on the order of three seconds.

Movie for encoding b11 includes a total of 20 clock images, of which 10 are at an upper portion and the other 10 are at a lower portion of display surface 11. Of these 20 clock images, only the second right clock image M1 in the bottom line is rotating. This rotation may either be a high-speed rotation of the clock hands or the rotation of the clock image itself. The other 19 clock images stay still to attract the attention of the subject to clock image M1.

In movie for encoding b12, a dotted line frame is added to enhance only the rotating clock image M2 and the other clock images disappear. This is to attract the attention of the subject to clock image M2 without using a linguistic instruction.

In step S64, another movie is displayed for Tc seconds as an interval in the memorization. Tc may be, for example, on the order of 60 seconds.

Display times Td, Te, and Tf in the second iteration in FIG. 7 may be the same as or different from times Ta, Tb, and Tc in the first iteration.

Such iteration of loop 1 causes the subject to unconsciously focus his/her attention on clock image M1 on the screen, thereby augmenting the memory.

After loop 1, PC 30 displays assessment movie b3 for recalling the memory for Tg seconds (S66). Time Tg may be, for example, on the order of five seconds. Assessment movie b3 shown in FIG. 7 includes clock image M3 instead of clock image M1. Stated differently, assessment movie b3, in which all of the 20 clock images stay still, is originally configured not to attract the attention only to a specific graphic.

A subject without memory impairment remembers the contents of the movie for encoding (movie for encoding b11 and movie for encoding b12) that such subject has viewed, and thus unconsciously focuses the eye gaze at the specific graphic or region on which his/her attention was focused in the movie for encoding. The specific graphic or region, which is a graphic or region that the subject has memorized from the movie for encoding, is still clock image M3 located at the same position as clock image M1 in FIG. 7.

In contrast, a subject with memory impairment cannot retain his/her memory or remember the contents, and thus tends to view randomly without unconsciously focusing the eye gaze at clock image M3.

Subsequently, to quantitatively assess the memory power, PC 30 sets, as a region of interest, a specific graphic or region in the assessment movie for recalling the memory and calculates, as a score of the memory power, % fixation duration Vb for which the region of interest is focused on (S67). Here, % fixation duration Vb may be the percentage (%) of the presence of gaze points on the region of clock image M3, which is the region of interest, in the distribution pattern of gaze points on the assessment movie.

PC 30 further determines whether % fixation duration Vb calculated is greater than threshold th1 (S68). When determining that % fixation duration Vb is greater than threshold th1, the subject is diagnosed as having no memory impairment (S69).

PC 30 further determines whether % fixation duration Vb calculated is lower than threshold th1 and greater than threshold th2 (S70). When determining that % fixation duration Vb is lower than threshold th1 and greater than threshold th2, the subject is diagnosed as having suspected memory impairment (S71).

PC 30 further determines whether % fixation duration Vb calculated is lower than threshold th2 (S72). When determining that % fixation duration Vb is lower than threshold th2, the subject is diagnosed as having memory impairment (S73).

Threshold th1 and threshold th2 can be appropriately set by calculating % fixation duration Vb of a plurality of subjects who are known beforehand whether having or not having memory impairment.

Note that FIG. 6 shows an example of outputting three diagnostic results of "having no memory impairment", "having suspected memory impairment", and "having memory impairment", but the diagnostic results are not limited to these. The diagnostic results thus may come in two types indicating the presence or absence of memory impairment, or may be four or more types indicating the presence or absence of memory impairment and the level of memory impairment.

Note that the flowchart of FIG. 6 may include, instead of steps S61 through S66, a step of obtaining a distribution map that represents the distribution of gaze points of a subject on the movie for cognitive assessment. The flowchart in which such steps are replaced by the step of obtaining the distribution map is easily implemented by PC 30 shown in FIG. 1B or may be implemented by PC 30 shown in FIG. 1A. Next, an example of the distribution map obtained in the first diagnostic process will be described.

FIG. 8 is a diagram showing an example distribution map obtained in the first diagnostic process performed on a subject without memory impairment and having an MMSE score of 30. FIG. 9 is a diagram showing an example distribution map obtained in the first diagnostic process performed on a subject with memory impairment and having an MMSE score of 14. Note that a perfect MMSE score is 30. The score 30 indicates that a person is cognitively unimpaired. The lower the score, the more severe cognitive impairment.

FIG. 8 and FIG. 9 each show an example in which a distribution map is superimposed on each of movie for encoding b11, movie for encoding b12, and assessment movie b3 to be displayed on display 35 of PC 30.

In movie for encoding b11 and movie for encoding b12, as shown in FIG. 8 and FIG. 9, gaze points of the subject concentrate on clock image M1. Movie for encoding b11 and movie for encoding b12 are movies for cognitive assessment each including: a first region including clock image M1 that is an image for encoding and displayed in a visually enhanced manner; and a plurality of second regions including still clock images that are normal images displayed without being enhanced. As shown in FIG. 8 and FIG. 9, the gaze points, on the movies for encoding, of the subjects with and without memory impairment are almost the same; their gaze points concentrate on clock image M1 in the first region.

The gaze point distribution, on assessment movie b3 shown in FIG. 8, of the subject without memory impairment exhibits a high % fixation duration of 81% for clock image M3 of the region of interest, as a result of the effect of the memorization.

In contrast, the gaze point distribution, on assessment movie b3 shown in FIG. 9, of the subject with memory impairment exhibits a % fixation duration of 0% for clock image M3 of the region of interest, without any effects of the memorization.

In the first diagnostic process, memory impairment is diagnosed by quantitatively assessing the memory level of a subject to obtain, as a score of the memory power, a % fixation duration for which the region of interest on the assessment movie for recalling the memory is focused on.

As described above, the cognitive impairment diagnostic device that performs the first diagnostic process includes: obtainer 41 that obtains a distribution map representing a distribution of gaze points of a subject on a movie for cognitive assessment; and diagnoser 39 that makes diagnosis of cognitive impairment, based on the distribution map. Here, the movie for cognitive assessment includes, in time order: a first movie including (i) a first region that includes an image for encoding that is displayed in a visually enhanced manner, and (ii) a plurality of second regions that include normal images that are displayed without being enhanced; and an assessment movie including (iii) a third region that includes an assessment image that is similar to the image for encoding and displayed at a same position as the image for encoding without being visually enhanced, and (iv) the plurality of second regions. Diagnoser 39 calculates, in a distribution map on the assessment movie, a percentage of fixation duration for which the third region is focused on, and makes diagnosis of suspected memory impairment when the percentage of fixation duration is lower than a threshold.

Also, the cognitive impairment diagnostic device that performs the first diagnostic process includes: display 10 including a display surface; imager 21 that captures an image of the eyes of a subject; detector 37 that detects, on the time series, gaze points of the subject on the display surface, on the basis of the image captured by imager 21; generator 38 that generates a distribution map representing a distribution of the gaze points detected by detector 37; and diagnoser 39 that makes diagnosis of cognitive impairment, on the basis of the distribution map. Here, diagnoser 39 causes display 10 to display a first movie that includes a first region including an image for encoding that is displayed in a visually enhanced manner, and a plurality of second regions including normal images that are displayed without being enhanced. Diagnoser 39 further causes display 10 to display an assessment movie that includes a third region including an assessment image that is similar to the image for encoding and displayed at a same position as the image for encoding without being visually enhanced, and the plurality of second regions. Then, diagnoser 39 calculates, in the distribution map on the assessment movie, the percentage of fixation duration for which the third region is focused on, and makes diagnosis of suspected memory impairment when the percentage of fixation duration is lower than a threshold.

With this, it is possible to provide a cognitive impairment diagnostic device capable of making a simple, low-cost, objective, quantitative, and versatile diagnosis of memory impairment among cognitive impairments. What is more, it is possible to make the diagnosis without using an instruction by text and instruction by voice. This enables the diagnosis to be made on a plurality of subjects having different mother languages. Further, memory impairment is diagnosed within a mere few minutes.

Here, the first movie may include a first movie for encoding and a second movie for encoding in time order, the first movie for encoding including the first region and the plurality of second regions, the second movie for encoding including the first region and not including the plurality of second regions.

With this, it is possible to augment the ability of the subject to remember the first region.

Here, the movie for cognitive assessment may include an other movie that is displayed between the first movie and the assessment movie.

With this, it is possible to more distinctively determine whether the subject is capable of retaining the memory power by providing a period of displaying another movie between the memorization and the assessment. This thus increases the reliability of the diagnostic result.

Here, the movie for cognitive assessment may include a movie in which the first movie and the other movie are repeated two or more times.

With this, it is possible to augment the ability of the subject to remember the first region by such repetition.

Also, the cognitive impairment diagnostic program that performs the first diagnostic process is a cognitive impairment diagnostic program executed by a computer that obtains a distribution map representing a distribution of gaze points of a subject on a movie for cognitive assessment, and makes diagnosis of cognitive impairment, based on the distribution map. Here, the movie for cognitive assessment includes, in time order: a first movie including (i) a first region that includes an image for encoding that is displayed in a visually enhanced manner, and (ii) a plurality of second regions that include normal images that are displayed without being enhanced; and an assessment movie including (iii) a third region that includes an assessment image that is similar to the image for encoding and displayed at a same position as the image for encoding without being visually enhanced, and (iv) the plurality of second regions. Such cognitive impairment diagnostic program causes the computer to execute: calculating, in a distribution map on the assessment movie, a percentage of fixation duration for which the third region is focused on; and making diagnosis of suspected memory impairment when the percentage of fixation duration is lower than a threshold.

Also, the cognitive impairment diagnostic program that performs the first diagnostic process is a cognitive impairment diagnostic program executed by a computer that includes: display 10 including a display surface; imager 21 that captures an image of the eyes of a subject; detector 37 that detects, on the time series, gaze points of the subject on the display surface, on the basis of the image captured by imager 21; a generator that generates a distribution map representing a distribution of the gaze points detected by detector 37; and diagnoser 39 that makes diagnosis of cognitive impairment, on the basis of the distribution map. Such cognitive impairment diagnostic program causes the computer to execute: causing display 10 to display a movie for cognitive assessment that includes a first region including an image for encoding that is displayed in a visually enhanced manner, and a plurality of second regions including normal images that are displayed without being enhanced; further causing display 10 to display an assessment movie that includes a third region including an assessment image that is similar to the image for encoding and displayed at a same position as the image for encoding without being visually enhanced, and the plurality of second regions; calculating, in the distribution map on the assessment movie, the percentage of fixation duration for which the third region is focused on, and making diagnosis of suspected memory impairment when the percentage of fixation duration is lower than a threshold.

### [2.2.1 Second Diagnostic Process (Example of Using Linguistic Instruction)]

Next, the second diagnostic process in step S20 shown in FIG. 5 in which a linguistic instruction is used will be described. The second diagnostic process uses the foregoing second characteristics to make diagnosis of aphasia.

FIG. 10 is a flowchart of an example of the second diagnostic process according to the embodiment. FIG. 11 is a diagram showing an example movie for cognitive assessment in the second diagnostic process in FIG. 10 in which a linguistic instruction is used.

In FIG. 10, PC 30 starts the detection of gaze points by detector 37 (S101), displays the movie for cognitive assessment on display surface 11 (S102), and calculates % fixation duration Vc for which the correct region is focused on in the movie for cognitive assessment (S103).

The movie for cognitive assessment here is a movie that includes a first region including a correct character image and a plurality of second regions including incorrect character images.

In an example shown in FIG. 11, movie for cognitive assessment c11 is displayed for Ta seconds, after which movie for cognitive assessment c12 is displayed for Tb seconds. Time Ta and time Tb may each be, for example, on the order of 5 seconds to 10 seconds.

Movie for cognitive assessment c11 includes a clock image at a central portion, around which four regions serving as alternatives are included. Movie for cognitive assessment c12 is different from movie for cognitive assessment c11 in that an instruction sentence is laid over the clock image at the central portion. The instruction sentence reads "What is the name of this?". The four regions around the instruction sentence include the first region including the correct character image " 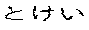 " (a Japanese word for "clock") a and the plurality of second regions including incorrect character images " 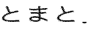 " (Japanese word for "tomato"), " 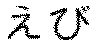 " (Japanese word for "shrimp"), and " 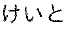 " (Japanese word for "wool yarn").

As described above, the movie for cognitive assessment includes, for example, a plurality of regions serving as alternatives that include an image of a known object, and correct and incorrect names of such object. The number of alternatives is not limited to a specific number. The movie for cognitive assessment also shows an instruction sentence that reads "What is the name of this?". The subject is prompted to gaze at the correct alternative by the instruction sentence.

Note that one movie for cognitive assessment c13 as shown in FIG. 12 may be displayed instead of two movie for cognitive assessments c11 and c12 shown in FIG. 11.

Further, PC 30 calculates % fixation duration Vc for which the first region that includes the correct character image is focused on, after displaying the movie for cognitive assessment (S103). Here, % fixation duration Vc may be the percentage (%) of the presence of gaze points in the first region in the distribution pattern of gaze points on the movie for cognitive assessment.

Next, PC 30 determines whether % fixation duration Vc calculated is greater than threshold th3 (S104). When determining that % fixation duration Vc is greater than threshold th3, the subject is diagnosed as having no aphasia (S105).

PC 30 further determines whether % fixation duration Vc calculated is lower than threshold th3 and greater than threshold th4 (S106). When determining that % fixation duration Vc is lower than threshold th3 and greater than threshold th4, the subject is diagnosed as having suspected aphasia (S107).

PC 30 further determines whether % fixation duration Vc calculated is lower than threshold th4 (S108). When determining that % fixation duration Vc is lower than threshold th4, the subject is diagnosed as having aphasia (S109).

Threshold th3 and threshold th4 can be appropriately set by calculating % fixation duration Vc of a plurality of subjects who are known beforehand whether having or not having aphasia.

Note that the flowchart of FIG. 10 may include, instead of steps S101 and S102, a step of obtaining a distribution map that represents the distribution of gaze points of a subject on the movie for cognitive assessment. The flowchart in which such steps are replaced by the step of obtaining the distribution map is easily implemented by PC 30 shown in FIG. 1B or may be implemented by PC 30 shown in FIG. 1A.

Note that FIG. 10 shows an example of outputting three diagnostic results of "having no aphasia", "having suspected aphasia", and "having aphasia", but the diagnostic results are not limited to these. The diagnostic results thus may come in two types indicating the presence or absence of aphasia, or may be four or more types indicating the presence or absence of aphasia and the level of aphasia.

Next, an example of the distribution map obtained in the second diagnostic process will be described.

FIG. 13 is a diagram showing an example distribution map obtained in the second diagnostic process performed on a subject without aphasia and having an MMSE score of 30. FIG. 14 is a diagram showing an example distribution map obtained in the second diagnostic process performed on a subject with aphasia and having an MMSE score of 14.

The gaze point distribution, on movie for cognitive assessment c12 shown in FIG. 13, of a subject without aphasia exhibits a high % fixation duration for which first region c111 that includes the correct character image is focused on.

In contrast, the gaze point distribution, on movie for cognitive assessment c12 shown in FIG. 14, of a subject with aphasia exhibits a low % fixation duration for which the first region that includes the correct character image is focused on.

When % fixation duration Vc for which the first region serving as the correct answer is focused on exceeds threshold th3, it can be determined that the answer to the instruction sentence is correct, that is, object naming ability is normal. Stated differently, a subject without aphasia is capable of focusing on the correct alternative " 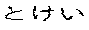 " at a rate of the threshold or greater. In contrast, a subject with aphasia has a relatively high rate of focusing on an alternative other than the correct answer. It is thus possible in the second diagnostic process to make diagnosis of whether a subject has aphasia and the level of aphasia, using as a measure, the % fixation duration for which the correct alternative is focused on.

### [2.2.2 Second Diagnostic Process (First Example Using No Linguistic Instruction)]

An example of the second diagnostic process in which a linguistic instruction is used has been described above. Next, a first example of using no linguistic instruction in the second diagnostic process will be described.

FIG. 15 is a diagram showing the first example of a movie for cognitive assessment in the second diagnostic process shown in FIG. 10 in which no linguistic instruction is used.

Movie for cognitive assessment c21 in FIG. 15, which shows an example display on display surface 11 for a subject, includes a correct region including characters having a meaning and incorrect regions including characters having no meaning. More specifically, movie for cognitive assessment c21 includes a plurality of characters arranged in matrix. The correct region corresponds to a row or column that includes characters having a meaning. Incorrect regions correspond to rows or columns that include characters having no meaning. The row that includes characters " 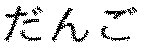 " (the name of a Japanese sweet) is the correct region. The other rows and columns are incorrect regions including no characters having a meaning.

FIG. 16 is a diagram showing an example assessment movie that corresponds to FIG. 15. FIG. 16 shows an example display on display 35 for the operator. In FIG. 16, correct region c211 serving as an assessment region is specified by a broken line frame compared to movie for cognitive assessment c21 shown in FIG. 15.

Next, an example gaze point distribution of a subject on movie for cognitive assessment c21 shown in FIG. 15 will be described.

FIG. 17 is a diagram showing an example distribution map obtained in the second diagnostic process that is performed, using FIG. 15, on a subject without aphasia and having an MMSE score of 30. FIG. 18 is a diagram showing an example distribution map obtained in the second diagnostic process that is performed, using FIG. 15, on a subject with aphasia and having an MMSE score of 14.

The gaze point distribution, on movie for cognitive assessment c21 shown in FIG. 17, of the subject without aphasia exhibits a high % fixation duration for which correct region c211 is focused on.

In contrast, the gaze point distribution, on movie for cognitive assessment c21 shown in FIG. 18, of the subject with aphasia tends to be random irrelevantly to correct region c211.

In movie for cognitive assessment c21, characters are arranged in matrix or in a grid pattern, in which "characters as a sequence of characters having a meaning" is present only in a specific portion. The other portions include no characters as a sequence of characters having a meaning when read from any directions.

When such characters in matrix are displayed, a subject without aphasia is capable of finding characters having a meaning therefrom. A % fixation duration of such subject focusing on the corresponding portion becomes automatically high.

Meanwhile, a subject with aphasia is incapable of finding characters as a sequence of characters having a meaning, and thus tends to view the entire screen randomly. A % fixation duration of such subject focusing on the specific portion thus does not become high relatively. In view of this, it is possible in the second diagnostic process to perform an objective diagnosis of aphasia, using as a measure, whether the % fixation duration for which a portion corresponding to the characters as a sequence of characters having a meaning (i.e., correct region) is focused on exceeds a threshold.

Characters including 3×3 hiragana characters are displayed on movie for cognitive assessment c21 shown in FIG. 15, in which characters having a meaning " 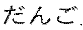 " is recognizable when the three characters in the bottom line are read from left to right. The other sequences of characters are characters basically having no meaning. While no text or voice is provided as an instruction sentence to prompt a subject to find " 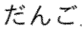 ", the % fixation duration of a cognitively unimpaired person without aphasia focusing on the characters as a sequence of characters having a meaning " 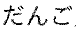 " automatically rises relatively.

In contrast, a subject with aphasia is incapable of recognizing such characters as a sequence of characters having a meaning and tends to view another region or view the entire screen randomly, with no rise in the % fixation duration for which " 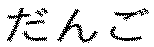 " is focused on.

### [2.2.3 Second Diagnostic Process (Second Example Using No Linguistic Instruction)]

Next, a second example of using no linguistic instruction in the second diagnostic process will be described.

FIG. 19 is a diagram showing the second example of a movie for cognitive assessment in the second diagnostic process shown in FIG. 10 in which no linguistic instruction is used. FIG. 20 is a diagram showing an example assessment movie that corresponds to FIG. 19.

Movie for cognitive assessment c31 shown in FIG. 19, which shows an example display on display surface 11 for a subject, includes a correct region including characters having a meaning and incorrect regions including characters having no meaning. More specifically, in movie for cognitive assessment c31, the row that includes characters " 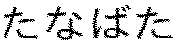 " (Japanese festival known as the Star Festival) is the correct region and the other rows and columns are incorrect regions including no characters having a meaning.

FIG. 20 shows an example display on display 35 for the operator. In FIG. 20, correct region c311 serving as an assessment region is specified by a broken line frame compared to movie for cognitive assessment c31 shown in FIG. 19.

Next, an example gaze point distribution of a subject on movie for cognitive assessment c31 shown in FIG. 19 will be described.

FIG. 21 is a diagram showing an example distribution map obtained in the second diagnostic process that is performed, using FIG. 20, on a subject without aphasia and having an MMSE score of 30. FIG. 22 is a diagram showing an example distribution map obtained in the second diagnostic process that is performed, using FIG. 20, on a subject with aphasia and having an MMSE score of 14.

The gaze point distribution on movie for cognitive assessment c31 shown in FIG. 21 of the subject without aphasia exhibits a high % fixation duration for which correct region c311 is focused on. In contrast, the gaze point distribution on movie for cognitive assessment c31 shown in FIG. 22 of the subject with aphasia tends to be random irrelevantly to correct region c311.

Characters including 4×4 hiragana characters are displayed in movie for cognitive assessment c31 shown in FIG. 19, in which characters having a meaning " 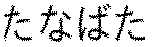 " is recognizable when the four characters in the second right column are read from top to bottom. The other sequences of characters are characters basically having no meaning. While no text or voice is provided as an instruction sentence to prompt a subject to find " 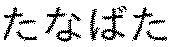 ", the % fixation duration of a cognitively unimpaired person without aphasia focusing on the characters " 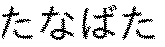 " having a meaning automatically rises relatively. In contrast, a subject with aphasia is incapable of recognizing such characters as a sequence of characters having a meaning and tends to view another region or view the entire screen randomly with no rise in the % fixation duration for which such portion is focused on.

As described above, cognitive impairment diagnostic device 1 that performs the second diagnostic process includes: obtainer 41 that obtains a distribution map representing a distribution of gaze points of a subject on a movie for cognitive assessment; and diagnoser 39 that makes diagnosis of cognitive impairment, based on the distribution map. Here, the movie for cognitive assessment includes: a first region including a correct image for cognitive assessment or an image for encoding; and a plurality of second regions including other images. Diagnoser 39 calculates, in the distribution map on the movie for cognitive assessment, a percentage of fixation duration for which the first region is focused on or a percentage of fixation duration for which at least one of the plurality of second regions is focused on, and makes diagnosis of whether cognitive impairment is present by comparing the percentage of fixation duration with a threshold.

Also, cognitive impairment diagnostic device 1 that performs the second diagnostic process includes: display 10 including a display surface; imager 21 that captures an image of the eyes of a subject; detector 37 that detects, on the time series, gaze points of the subject on the display surface, on the basis of the image captured by imager 21; a generator that generates a distribution map representing a distribution of the gaze points detected by detector 37; and diagnoser 39 that makes diagnosis of cognitive impairment, on the basis of the distribution map. Here, diagnoser 39 causes display 10 to display a movie for cognitive assessment that includes a first region including a correct image for cognitive assessment or an image for encoding, and a plurality of second regions including other images. Then, diagnoser 39 calculates, in the distribution map on the movie for cognitive assessment, the percentage of fixation duration for which the first region is focused on or the percentage of fixation duration for which at least one of the second regions is focused on, and makes diagnosis of the presence or absence of cognitive impairment by comparing the percentage of fixation duration with a threshold.

With this, it is possible to provide a cognitive impairment diagnostic device capable of making a simple, low-cost, objective, quantitative, and versatile diagnosis of cognitive impairment. It is further possible to diagnose cognitive impairment within a mere few seconds to a few tens of second.

Also, cognitive impairment diagnostic device 1 that performs the second diagnostic process includes: obtainer 41 that obtains a distribution map representing a distribution of gaze points of a subject on a movie for cognitive assessment; and diagnoser 39 that makes diagnosis of cognitive impairment, based on the distribution map. Here, the movie for cognitive assessment includes: the first region including a correct character image; and the plurality of second regions including incorrect character images. Diagnoser 39 calculates, in the distribution map on the movie for cognitive assessment, the percentage of fixation duration for which the first region is focused on, and makes diagnosis of suspected aphasia when the percentage of fixation duration is lower than the threshold.

Also, cognitive impairment diagnostic device 1 that performs the second diagnostic process includes: display 10 including a display surface; imager 21 that captures an image of the eyes of a subject; detector 37 that detects, on the time series, gaze points of the subject on the display surface, on the basis of the image captured by imager 21; a generator that generates a distribution map representing a distribution of the gaze points detected by detector 37; and diagnoser 39 that makes diagnosis of cognitive impairment, on the basis of the distribution map. Here, diagnoser 39 causes display 10 to display a movie for cognitive assessment that includes a first region including correct characters and a plurality of second regions including incorrect characters. Then, diagnoser 39 calculates, in the distribution map on the movie for cognitive assessment, the percentage of fixation duration for which the first region is focused on, and makes diagnosis of suspected aphasia when the percentage of fixation duration is lower than a threshold.

With this, it is possible to provide a cognitive impairment diagnostic device capable of making a simple, low-cost, objective, quantitative, and versatile diagnosis of aphasia among cognitive impairments. It is further possible to diagnose cognitive impairment within a mere few seconds to a few tens of second.

Here, the movie for cognitive assessment may include: an image region including an image representing an object that is known; a question region including a question sentence asking a name of the object; the first region that is a correct region including character image indicating the name of the object; and the plurality of second regions that are incorrect regions including character images other than the name of the object.

With this, it is possible to make diagnosis of aphasia relating to the name of an object, using a question sentence as a linguistic instruction.

Here, the movie for cognitive assessment may include: the correct region including characters having a meaning; and incorrect regions including characters having no meaning. With this, it is possible to make diagnosis of aphasia without using an instruction sentence and a voice instruction.

Here, the movie for cognitive assessment may include a plurality of characters arranged in matrix, the correct region may correspond to a row or a column that is formed of the characters having the meaning, and the incorrect regions may correspond to rows or columns formed of the characters having no meaning.

With this, it is possible to make diagnosis of aphasia, using a simplified movie for cognitive assessment, without using an instruction sentence and a voice instruction.

The cognitive impairment diagnostic program that performs the second diagnostic process is a cognitive impairment diagnostic program executed by a computer that obtains a distribution map representing a distribution of gaze points of a subject on a movie for cognitive assessment and makes diagnosis of cognitive impairment on the basis of the distribution map. Here, the movie for cognitive assessment includes a first region including a correct image for cognitive assessment or an image for encoding, and a plurality of second regions including other images. Such cognitive impairment diagnostic program causes the computer to execute: calculating, in the distribution map on the movie for cognitive assessment, the percentage of fixation duration for which the first region is focused on or the percentage of fixation duration for which at least one of the second regions is focused on; and making diagnosis of the presence or absence of cognitive impairment by comparing the percentage of fixation duration with a threshold.

Also, the cognitive impairment diagnostic program that performs the second diagnostic process is a cognitive impairment diagnostic program executed by a computer that includes: display 10 including a display surface; imager 21 that captures an image of the eyes of a subject; detector 37 that detects, on the time series, gaze points of the subject on the display surface, on the basis of the image captured by imager 21; a generator that generates a distribution map representing a distribution of the gaze points detected by detector 37; and diagnoser 39 that makes diagnosis of cognitive impairment, on the basis of the distribution map. Such cognitive impairment diagnostic program causes the computer to execute: causing display 10 to display a movie for cognitive assessment that includes a first region including a correct image for cognitive assessment or an image for encoding, and a plurality of second regions including other images; calculating, in the distribution map on the movie for cognitive assessment, the percentage of fixation duration for which the first region is focused on or the percentage of fixation duration for which at least one of the second regions is focused on; and making diagnosis of the presence or absence of cognitive impairment by comparing the percentage of fixation duration with a threshold.

The cognitive impairment diagnostic program that performs the second diagnostic process is a cognitive impairment diagnostic program executed by a computer that obtains a distribution map representing a distribution of gaze points of a subject on a movie for cognitive assessment and makes diagnosis of cognitive impairment on the basis of the distribution map. Here, the movie for cognitive assessment includes a first region including correct characters and a plurality of second regions including incorrect characters. Such cognitive impairment diagnostic program causes the computer to execute: calculating, in the distribution map on the movie for cognitive assessment, the percentage of fixation duration for which the first region is focused on, and making diagnosis of suspected aphasia when the percentage of fixation duration is lower than a threshold.

Also, the cognitive impairment diagnostic program that performs the second diagnostic process is a cognitive impairment diagnostic program executed by a computer that includes: display 10 including a display surface; imager 21 that captures an image of the eyes of a subject; detector 37 that detects, on the time series, gaze points of the subject on the display surface, on the basis of the image captured by imager 21; a generator that generates a distribution map representing a distribution of the gaze points detected by detector 37; and diagnoser 39 that makes diagnosis of cognitive impairment, on the basis of the distribution map. Such cognitive impairment diagnostic program causes the computer to execute: causing display 10 to display a movie for cognitive assessment that includes a first region including correct characters and a plurality of second regions including incorrect characters; calculating, in the distribution map on the movie for cognitive assessment, the percentage of fixation duration for which the first region is focused on; and making diagnosis of suspected aphasia when the percentage of fixation duration is lower than a threshold.

### [2.3 Third Diagnostic Process]

Next, the third diagnostic process in step S30 shown in FIG. 5 will be described. The third diagnostic process uses the foregoing third characteristics to make diagnosis of cognitive impairment.

FIG. 23 is a flowchart of an example third diagnostic process according to the embodiment. FIG. 24 is a diagram showing an example movie for cognitive assessment used in the third diagnostic process shown in FIG. 23.

In FIG. 23, PC 30 starts the detection of gaze points by detector 37 (S201), displays the movie for cognitive assessment on display surface 11 (S202), and calculates % fixation duration Va for which an incorrect region is focused on in the movie for cognitive assessment (S203).

The movie for cognitive assessment here is a movie that includes a first region including a correct image and a plurality of second regions including incorrect images. The movie for cognitive assessment is, for example, a movie that includes: a first region including a correct image that represents a first graphic; an instruction sentence region that includes an instruction sentence for inducing a subject to directly focus on the first graphic; second regions including incorrect images that represent graphics similar to the first graphic; and another second region including an incorrect image that represents a graphic not similar to the first graphic.

An example of FIG. 24 shows movie for cognitive assessment a1 that is displayed on display surface 11 for a subject. Such movie for cognitive assessment a1 includes an instruction sentence that reads "Focus on the hexagon", around which the graphics of a pentagon, a hexagon, a heptagon, and a triangle are included.

FIG. 25 is a diagram showing an example assessment movie used in the third diagnostic process shown in FIG. 23. An example of FIG. 25 shows movie for cognitive assessment a1 serving as the assessment movie to be displayed on display 35 of PC 30. Movie for cognitive assessment a1 in FIG. 25 includes: a first region including correct image a11 showing the hexagon as the first graphic; instruction sentence region a12 that reads "Focus on the hexagon"; and a plurality of second regions including incorrect images. The plurality of second regions include: a second region including an incorrect image showing the pentagon that is similar to the first graphic; a second region including an incorrect image showing the heptagon that is similar to the first graphic; and a second region including irrelevant incorrect image a13 showing the triangle that is not similar to the first graphic.

Further, PC 30 calculates % fixation duration Va for which incorrect regions that include incorrect images are focused on after displaying the movie for cognitive assessment (S203). Here, % fixation duration Va may be the percentage (%) of the presence of gaze points in the second region in the distribution pattern of gaze points on the movie for cognitive assessment. Also, an incorrect region for which % fixation duration Va is calculated may be any one of the second region including irrelevant incorrect image a13, instruction sentence region a12, or a region including a combination of the second region of irrelevant incorrect image a13 and instruction sentence region a12.

Next, PC 30 determines whether % fixation duration Va calculated is greater than threshold th5 (S204). When determining that % fixation duration Va is greater than threshold th5, the subject is diagnosed as having cognitive impairment (S205).

PC 30 further determines whether % fixation duration Va calculated is lower than threshold th5 and greater than threshold th6 (S206). When determining that % fixation duration Va is lower than threshold th5 and greater than threshold th6, the subject is diagnosed as having suspected cognitive impairment (S207).

PC 30 further determines whether % fixation duration Va calculated is lower than threshold th6 (S208). When determining that % fixation duration Va is lower than threshold th6, the subject is diagnosed as having no cognitive impairment (S209).

Threshold th5 and threshold th6 can be appropriately set by calculating % fixation duration Va of a plurality of subjects who are known beforehand whether having or not having cognitive impairment.

Note that the flowchart of FIG. 23 may include, instead of steps S201 and S202, a step of obtaining a distribution map that represents the distribution of gaze points of a subject on the movie for cognitive assessment. The flowchart in which such steps are replaced by the step of obtaining the distribution map is easily implemented by PC 30 shown in FIG. 1B or may be implemented by PC 30 shown in FIG. 1A.

FIG. 26 is a diagram showing an example distribution map in the third diagnostic process that is performed, using FIG. 24, on a subject without cognitive impairment. As shown in the diagram, the subject without cognitive impairment selects and focuses on correct image a11 from the alternatives in accordance with the instruction sentence shown on the screen. The % fixation duration for which s/he focuses on incorrect images is low. In particular, the % fixation duration for which irrelevant incorrect image a13 is focused on is extremely low.

In the third diagnostic process, the cognitive function of the subject is assessed, using as a measure, not the % fixation duration for which the correct image is focused on, but the % fixation duration for which an incorrect image is focused on and the % fixation duration for which the instruction sentence region is focused on. Stated differently, the % fixation duration for which the instruction sentence region is focused on and the % fixation duration for which incorrect images are focused on in the movie for cognitive assessment are inversely correlated with the cognitive function of the subject. It is thus possible to assess cognitive function, using these % fixation duration as a measure.

In particular, the % fixation duration for which "irrelevant incorrect image" is focused on is inversely correlated with the cognitive function of the subject among the % fixation duration for which incorrect images are focused on. Suppose, for example, that four alternative are presented, one of which is a correct image and other three are incorrect images. Also suppose that one of the three incorrect images that is no similar to the correct image is regarded as an irrelevant incorrect image. Stated differently, the irrelevant incorrect image is an alternative that can be determined to be clearly incorrect by a person with normal cognitive function. While the % fixation duration for which the correct image is focused on is positively correlated with the cognitive function of a subject, the % fixation duration for which the irrelevant incorrect image is focused on is negatively correlated with the cognitive function of a subject.

The movie for cognitive assessment in FIG. 24 shows four images (pentagon, hexagon, heptagon, and triangle) as the alternatives as well as the instruction sentence that reads "Focus on the hexagon". Here, the hexagon is "correct image" and the triangle is "irrelevant incorrect image" in this case. The pentagon and the heptagon are similar to the hexagon serving as the correct answer, and thus cause confusion in selecting the correct answer, but the triangle cannot be confusing for a cognitively unimpaired person.

FIG. 27 is a diagram showing a correlation between an MMSE score and the percentage of fixation duration for which the instruction sentence region in FIG. 25 is focused on in the third diagnostic process. FIG. 28 is a diagram showing a correlation between an MMSE score and the percentage of fixation duration for which the irrelevant incorrect image in FIG. 25 is focused on in the third diagnostic process.

As shown in FIG. 27 and FIG. 28, the % fixation duration for which instruction sentence region a12 is focused on and the % fixation duration for which irrelevant incorrect image a13 is focused on are both negatively correlated with the cognitive functioning score (MMSE score) of subjects from the standpoint of statistical significance.

On the basis of the above, the third diagnostic process assesses the cognitive function of a subject, using as a measure, not the % fixation duration for which the correct image is focused on, but the % fixation duration for which an incorrect image is focused on and the % fixation duration for which the instruction sentence region is focused on.

As described above, cognitive impairment diagnostic device 1 that performs the third diagnostic process includes: obtainer 41 that obtains a distribution map representing a distribution of gaze points of a subject on a movie for cognitive assessment; and diagnoser 39 that makes diagnosis of cognitive impairment, based on the distribution map. Here, the movie for cognitive assessment includes a first region including a correct image and a plurality of second regions including incorrect images. Diagnoser 39 calculates, in the distribution map on the movie for cognitive assessment, a percentage of fixation duration for which a region other than the first region is focused on, and makes diagnosis of suspected cognitive impairment when the percentage of fixation duration is higher than the threshold.

Also, cognitive impairment diagnostic device 1 that performs the third diagnostic process includes: display 10 including a display surface; imager 21 that captures an image of the eyes of a subject; detector 37 that detects, on the time series, gaze points of the subject on the display surface, on the basis of the image captured by imager 21; a generator that generates a distribution map representing a distribution of the gaze points detected by detector 37; and diagnoser 39 that makes diagnosis of cognitive impairment, on the basis of the distribution map. Here, diagnoser 39 causes display 10 to display a movie for cognitive assessment that includes a first region including a correct image and a plurality of second regions including incorrect images. Then, diagnoser 39 calculates, in the distribution map on the movie for cognitive assessment, the percentage of fixation duration for which a region other than the first region is focused on, and makes diagnosis of suspected cognitive impairment when the percentage of fixation duration is higher than a threshold.

With this, it is possible to provide a cognitive impairment diagnostic device capable of making a simple, low-cost, objective, quantitative, and versatile diagnosis of cognitive impairment. What is more, cognitive impairment is diagnosed, using as a measure, the percentage of fixation duration for which an incorrect image is focused on. It is further possible to diagnose cognitive impairment within a mere few seconds to a few tens of second.

Here, the movie for cognitive assessment may include: an instruction sentence region including an instruction sentence inducing a direct focus on a first graphic; the first region including the correct image representing the first graphic; a second region including an incorrect image representing a graphic similar to the first graphic, the second region being included in the plurality of second regions; and an other second region including an incorrect image representing a graphic not similar to the first graphic, the other second region being included in the plurality of second regions. Here, diagnoser 39 calculates a percentage of fixation duration for which the instruction sentence region or the other second region is focused on.

With this, it is possible to diagnose cognitive impairment, using as a measure, the % fixation duration for which the instruction sentence region or the other second region is focused on.

Also, the cognitive impairment diagnostic program that performs the third diagnostic process is a cognitive impairment diagnostic program executed by a computer that obtains a distribution map representing a distribution of gaze points of a subject on a movie for cognitive assessment and makes diagnosis of cognitive impairment on the basis of the distribution map. Here, the movie for cognitive assessment includes a first region including a correct image and a plurality of second regions including incorrect images. Such cognitive impairment diagnostic program causes the computer to execute: calculating, in the distribution map on the movie for cognitive assessment, the percentage of fixation duration for which a region other than the first region is focused on; and making diagnosis of suspected cognitive impairment when the percentage of fixation duration is higher than a threshold.

Also, the cognitive impairment diagnostic program that performs the third diagnostic process is a cognitive impairment diagnostic program executed by a computer that includes: display 10 including a display surface; imager 21 that captures an image of the eyes of a subject; detector 37 that detects, on the time series, gaze points of the subject on the display surface, on the basis of the image captured by imager 21; a generator that generates a distribution map representing a distribution of the gaze points detected by detector 37; and diagnoser 39 that makes diagnosis of cognitive impairment, on the basis of the distribution map. Such cognitive impairment diagnostic program causes the computer to execute: causing display 10 to display a movie for cognitive assessment that includes a first region including a correct image, and a plurality of second regions including incorrect images; calculating, in the distribution map on the movie for cognitive assessment, the percentage of fixation duration for which a region other than the first region is focused on; and making diagnosis of suspected cognitive impairment when the percentage of fixation duration is higher than a threshold.

Note that cognitive impairment diagnostic device 1 may be configured to perform processing simultaneously on a plurality of subjects. In this case, imager 21 is simply required to capture images of a plurality of subjects, detector 37 is simply required to detect gaze points of each of the subjects, generator 38 is simply required to generate a distribution map for each of the subjects, and diagnoser 39 is simply required to make diagnosis on each of the subjects. When configured to perform processing simultaneously on a plurality of subjects, cognitive impairment diagnostic device 1 may include a plurality of imaging devices 20. In this case, one imaging device 20 may be used for one subject or for a plurality of subjects. This enables cognitive impairment diagnostic device 1 to further increase the efficiency in a mass medical examination.

Note that the foregoing embodiment and variation thereof are intended to illustrate the technology according to the present invention, and therefore do not limit the scope of the appended Claims. The invention is defined by the appended claims.

### [Industrial Applicability]

The present invention is applicable for use as a cognitive impairment diagnostic device and a cognitive impairment diagnostic program for diagnosis of cognitive impairment.

### [Reference Signs List]

- 1: cognitive impairment diagnostic device
- 10: display
- 11: display surface
- 20: imaging device
- 21: imager
- 22, 23: camera
- 24: light source unit
- 25, 26: light source
- 30: PC
- 31: processor
- 32: storage
- 33: inputter
- 34: outputter
- 35: display
- 36: interface
- 37: detector
- 38: generator
- 39: diagnoser
- 300: movie data for cognitive assessment
- 301: first movie data
- 302: second movie data
- 303: third movie data
- 310: case characteristics data
- 311: first characteristics data
- 312: second characteristics data
- 313: third characteristics data
- 320: program
- 321: cognitive impairment diagnostic program
- 322: gaze point data
- 323: distribution map data
- 324: database

## Claims

1. A cognitive impairment diagnostic device (1) comprising:
a control unit (30) comprising and configured to control
a display configured for displaying a movie for cognitive assessment to a subject in order to generate a distribution map representing a distribution of gaze points of the subject on the movie for cognitive assessment,
an obtainer (41) configured to obtain said distribution map representing a distribution of gaze points of the subject on the movie for cognitive assessment; and
a diagnoser (39) configured to make a diagnosis of cognitive impairment, based on the distribution map,
wherein the movie for cognitive assessment includes, in time order:
a first movie including (i) a first region that includes an image for encoding that is displayed in a visually enhanced manner so as to attract the attention of the subject and help focus the subject's eye gaze on this first region, and (ii) a plurality of second regions that include normal images wherein a normal image is the same image as the image for encoding except that it isdisplayed without being enhanced; and
an assessment movie including (iii) a third region that includes an assessment image displayed at a same position as the image for encoding and that is similar to the image for encoding in that it is a normal image which is displayed without being visually enhanced, and (iv) the plurality of second regions, and
the diagnoser is further configured to:
calculate, in a distribution map on the assessment movie, a percentage of fixation duration for which the third region is focused on; and
make a diagnosis of suspected memory impairment when the percentage of fixation duration is lower than a threshold.

2. The cognitive impairment diagnostic device (1) according to claim 1,
wherein the first movie includes a first movie for encoding and a second movie for encoding in time order, the first movie for encoding including the first region and the plurality of second regions, the second movie for encoding including the first region and not including the plurality of second regions.

3. The cognitive impairment diagnostic device (1) according to claim 1 or 2,
wherein the movie for cognitive assessment includes an other movie that is displayed between the first movie and the assessment movie.

4. The cognitive impairment diagnostic device (1) according to claim 3,
wherein the movie for cognitive assessment includes a movie in which the first movie and the other movie are repeated two or more times.

5. A cognitive impairment diagnostic program (321) executed by a computer comprising a control unit (30) configured to
display a movie for cognitive assessment to a subject in order to generate a distribution map representing a distribution of gaze points of the subject on the movie for cognitive assessment,
obtain said distribution map representing a distribution of gaze points of a subject on the movie for cognitive assessment, and
make diagnosis of cognitive impairment, based on the distribution map,
wherein the movie for cognitive assessment includes, in time order:
a first movie including (i) a first region that includes an image for encoding that is displayed in a visually enhanced manner so as to attract the attention of the subject and help focus the subject's eye gaze on this first region, and (ii) a plurality of second regions that include normal images wherein a normal image is the same image as the image for encoding except that it is displayed without being enhanced; and
an assessment movie including (iii) a third region that includes an assessment image displayed at a same position as the image for encoding and that is similar to the image for encoding in that it is a normal image which is displayed without being visually enhanced, and (iv) the plurality of second regions, and
the cognitive impairment diagnostic program causing the computer to execute:
calculating, in a distribution map on the assessment movie, a percentage of fixation duration for which the third region is focused on; and
making diagnosis of suspected memory impairment when the percentage of fixation duration is lower than a threshold.

## Patentansprüche

1. Vorrichtung (1) zur Diagnose kognitiver Störungen, umfassend:
eine Steuereinheit (30), die Folgendes umfasst und dazu konfiguriert ist, Folgendes zu steuern:
eine Anzeige, die dazu konfiguriert ist, einen Film zur kognitiven Beurteilung für einen Probanden anzuzeigen, um eine Verteilungskarte zu generieren, die eine Verteilung von Blickpunkten des Probanden in dem Film zur kognitiven Beurteilung darstellt,
eine Abrufeinrichtung (41), die dazu konfiguriert ist, die Verteilungskarte abzurufen, die eine Verteilung von Blickpunkten des Probanden in dem Film zur kognitiven Beurteilung darstellt; und
eine Diagnoseeinrichtung (39), die dazu konfiguriert ist, basierend auf der Verteilungskarte eine Diagnose kognitiver Störung zu stellen,
wobei der Film zur kognitiven Beurteilung in zeitlicher Reihenfolge Folgendes umfasst:
einen ersten Film, der (i) eine erste Region, die ein Bild zur Codierung umfasst, das in einer visuell verbesserten Weise angezeigt wird, um die Aufmerksamkeit des Probanden zu erregen und zu helfen, den Blick des Probanden auf die erste Region zu fokussieren, und (ii) eine Mehrzahl von zweiten Regionen umfasst, die normale Bilder umfassen, wobei ein normales Bild dasselbe Bild wie das Bild zur Codierung ist, jedoch ohne Verbesserung angezeigt wird; und
einen Beurteilungsfilm, der (iii) eine dritte Region, die ein Beurteilungsbild umfasst, das an einer gleichen Position wie das Bild zur Codierung angezeigt wird und das dem Bild zur Codierung insofern ähnelt, als es ein normales Bild ist, das ohne visuelle Verbesserung angezeigt wird, und (iv) die Mehrzahl von zweiten Regionen umfasst, und
die Diagnoseeinrichtung ferner zu Folgendem konfiguriert ist:
Berechnen eines Prozentsatzes einer Fixationsdauer, für den die dritte Region fokussiert wird, in einer Verteilungskarte in dem Beurteilungsfilm; und
Stellen einer Diagnose einer vermuteten Gedächtnisstörung, wenn der Prozentsatz der Fixationsdauer unter einem Schwellenwert liegt.

2. Vorrichtung (1) zur Diagnose kognitiver Störungen nach Anspruch 1,
wobei der erste Film einen ersten Film zur Codierung und einen zweiten Film zur Codierung in zeitlicher Reihenfolge umfasst, wobei der erste Film zur Codierung die erste Region und die Mehrzahl von zweiten Regionen umfasst, der zweite Film zur Codierung die erste Region, aber nicht die Mehrzahl von zweiten Regionen umfasst.

3. Vorrichtung (1) zur Diagnose kognitiver Störungen nach Anspruch 1 oder 2,
wobei der Film zur kognitiven Beurteilung einen weiteren Film umfasst, der zwischen dem ersten Film und dem Beurteilungsfilm angezeigt wird.

4. Vorrichtung (1) zur Diagnose kognitiver Störungen nach Anspruch 3,
wobei der Film zur kognitiven Beurteilung einen Film umfasst, in dem der erste Film und der weitere Film zweimal oder mehrmals wiederholt werden.

5. Programm (321) zur Diagnose kognitiver Störungen, das von einem Computer ausgeführt wird, der eine Steuereinheit (30) umfasst, die zu Folgendem konfiguriert ist:
Anzeigen eines Films zur kognitiven Beurteilung für einen Probanden, um eine Verteilungskarte zu generieren, die eine Verteilung von Blickpunkten des Probanden in dem Film zur kognitiven Beurteilung darstellt,
Abrufen der Verteilungskarte, die eine Verteilung von Blickpunkten eines Probanden in dem Film zur kognitiven Beurteilung darstellt, und
Stellen einer Diagnose kognitiver Störung basierend auf der Verteilungskarte,
wobei der Film zur kognitiven Beurteilung in zeitlicher Reihenfolge Folgendes umfasst:
einen ersten Film, der (i) eine erste Region, die ein Bild zur Codierung umfasst, das in einer visuell verbesserten Weise angezeigt wird, um die Aufmerksamkeit des Probanden zu erregen und zu helfen, den Blick des Probanden auf die erste Region zu fokussieren, und (ii) eine Mehrzahl von zweiten Regionen umfasst, die normale Bilder umfassen, wobei ein normales Bild dasselbe Bild wie das Bild zur Codierung ist, jedoch ohne Verbesserung angezeigt wird; und
einen Beurteilungsfilm, der (iii) eine dritte Region, die ein Beurteilungsbild umfasst, das an einer gleichen Position wie das Bild zur Codierung angezeigt wird und das dem Bild zur Codierung insofern ähnelt, als es ein normales Bild ist, das ohne visuelle Verbesserung angezeigt wird, und (iv) die Mehrzahl von zweiten Regionen umfasst, und
wobei das Programm zur Diagnose kognitiver Störungen den Computer veranlasst, Folgendes auszuführen:
Berechnen eines Prozentsatzes einer Fixationsdauer, für den die dritte Region fokussiert wird, in einer Verteilungskarte in dem Beurteilungsfilm; und
Stellen einer Diagnose einer vermuteten Gedächtnisstörung, wenn der Prozentsatz der Fixationsdauer unter einem Schwellenwert liegt.

## Revendications

1. Dispositif de diagnostic des troubles cognitifs (1) comprenant :
une unité de commande (30) comprenant et configurée pour commander
un écran configuré pour afficher un film destiné à l'évaluation cognitive à un sujet afin de générer une carte de distribution représentant la répartition des points de regard du sujet sur le film destiné à l'évaluation cognitive,
un dispositif d'acquisition (41) configuré pour acquérir ladite carte de distribution représentant une distribution des points de regard du sujet sur le film destiné à l'évaluation cognitive ; et
un dispositif de diagnostic (39) configuré pour établir un diagnostic de troubles cognitifs, sur la base de la carte de distribution,
dans lequel le film destiné à l'évaluation cognitive comprend, dans l'ordre chronologique :
un premier film comprenant (i) une première région qui comprend une image à encoder affichée de manière visuellement mise en valeur afin d'attirer l'attention du sujet et d'aider à focaliser le regard du sujet sur cette première région, et (ii) une pluralité de deuxièmes régions qui comprennent des images normales, une image normale étant la même image que l'image à encoder, à l'exception qu'elle est affichée sans être mise en valeur ; et
un film d'évaluation comprenant (iii) une troisième région qui comprend une image d'évaluation affichée à la même position que l'image à encoder et qui est similaire à l'image à encoder en ce qu'il s'agit d'une image normale qui est affichée sans être mise en valeur visuellement, et (iv) la pluralité de deuxièmes régions, et
le dispositif de diagnostic est en outre configuré pour :
calculer, dans une carte de distribution sur le film d'évaluation, un pourcentage de durée de fixation pendant lequel la troisième région est focalisée ; et
établir un diagnostic de troubles de la mémoire présumés lorsque le pourcentage de durée de fixation est inférieur à un seuil.

2. Dispositif de diagnostic des troubles cognitifs (1) selon la revendication 1,
dans lequel le premier film comprend un premier film à encoder et un deuxième film à encoder dans l'ordre chronologique, le premier film à encoder comprenant la première région et la pluralité de deuxièmes régions, le deuxième film à encoder comprenant la première région et ne comprenant pas la pluralité de deuxièmes régions.

3. Dispositif de diagnostic des troubles cognitifs (1) selon la revendication 1 ou 2,
dans lequel le film destiné à l'évaluation cognitive comprend un autre film qui est affiché entre le premier film et le film d'évaluation.

4. Dispositif de diagnostic des troubles cognitifs (1) selon la revendication 3,
dans lequel le film destiné à l'évaluation cognitive comprend un film dans lequel le premier film et l'autre film sont répétés deux fois ou plus.

5. Programme de diagnostic des troubles cognitifs (321) exécuté par un ordinateur comprenant une unité de commande (30) configurée pour
afficher un film d'évaluation cognitive à un sujet afin de générer une carte de distribution représentant la répartition des points de regard du sujet sur le film d'évaluation cognitive,
obtenir ladite carte de distribution représentant une distribution des points de regard d'un sujet sur le film destiné à l'évaluation cognitive, et
établir un diagnostic de troubles cognitifs, sur la base de la carte de distribution,
dans lequel le film destiné à l'évaluation cognitive comprend, dans l'ordre chronologique :
un premier film comprenant (i) une première zone qui comprend une image à encoder affichée de manière visuellement mise en valeur afin d'attirer l'attention du sujet et d'aider à focaliser son regard sur cette première zone, et (ii) une pluralité de deuxièmes zones qui comprennent des images normales, une image normale étant la même image que l'image à encoder, à l'exception qu'elle est affichée sans avoir été mise en valeur ; et
un film d'évaluation comprenant (iii) une troisième région qui comprend une image d'évaluation affichée à la même position que l'image à encoder et qui est similaire à l'image à encoder en ce qu'il s'agit d'une image normale qui est affichée sans être mise en valeur visuellement, et (iv) la pluralité de deuxièmes régions, et
le programme de diagnostic des troubles cognitifs amenant l'ordinateur à exécuter :
le calcul, dans une carte de distribution sur le film d'évaluation, d'un pourcentage de durée de fixation pendant lequel la troisième région est focalisée ; et
établir un diagnostic de troubles de la mémoire présumés lorsque le pourcentage de durée de fixation est inférieur à un seuil.
